Europäisches Patentamt

European Patent Office

Office européen des brevets

(11) Veröffentlichungsnummer: **0 123 307**
**B1**

(12) **EUROPÄISCHE PATENTSCHRIFT**

(45) Veröffentlichungstag der Patentschrift: **06.06.90**

(21) Anmeldenummer: **84104546.1**

(22) Anmeldetag: **21.04.84**

(51) Int. Cl.⁵: **A 61 K 37/02**, C 07 K 15/00, C 07 K 15/06

(54) Gewebeprotein PP4, Verfahren zu seiner Gewinnung sowie seine Verwendung.

(30) Priorität: **26.04.83 DE 3315000**

(43) Veröffentlichungstag der Anmeldung:
**31.10.84 Patentblatt 84/44**

(45) Bekanntmachung des Hinweises auf die Patenterteilung:
**06.06.90 Patentblatt 90/23**

(84) Benannte Vertragsstaaten:
**AT BE CH DE FR GB IT LI LU NL SE**

(56) Entgegenhaltungen:
EP-A-0 009 715
EP-A-0 029 191
EP-A-0 033 000
EP-A-0 037 963
EP-A-0 060 491
DE-A-2 640 387
DE-A-2 720 704

CHEMICAL ABSTRACTS, Band 86, 1977, Seite 189, Nr. 135224x, Columbus, Ohio, US; H.BOHN et al.: "Isolation and characterization of a new tissue protein (PP7) from human placenta"

(73) Patentinhaber: **BEHRINGWERKE Aktiengesellschaft**
**Postfach 1140**
**D-3550 Marburg 1 (DE)**

(72) Erfinder: **Bohn, Hans, Dr.**
**Oberer Eichweg 26**
**D-3550 Marburg (DE)**

(74) Vertreter: **Klein, Otto, Dr. et al**
**Hoechst AG Zentrale Patentabteilung Postfach 80 03 20**
**D-6230 Frankfurt am Main 80 (DE)**

# EP 0 123 307 B1

**Beschreibung**

Die Erfindung betrifft ein Gewebeprotein, das als $PP_4$ bezeichnet wird, sowie ein Verfahren zu seiner Gewinnung.

$PP_4$ kann verwendet werden, um Antiseren herzustellen, die dazu dienen können, $PP_4$ in Körperflüssigkeiten nachzuweisen und zu bestimmen, um Erkrankungen bestimmter Organe zu erkennen, als "Marker" einen Krankheitsverlauf zu überwachen oder eine Therapie zu kontrollieren.

Gegenstand der Erfindung ist das Protein $PP_4$, gekennzeichnet durch

a) eine elektrophoretische Beweglichkeit im Bereich zwischen der von $\alpha_1$- und $\alpha_2$-Globulinen;

b) einen isoelektrischen Punkt von $4,85\pm0,15$;

c) einen Sedimentationskoeffizienten $s_{20,w}^0$ von $3,3\pm0,2S$;

d) ein im Natriumdodecylsulfat (SDS)-haltigen Polyacrylamidgel bestimmtes Molekulargewicht von $35.000\pm5.000$;

e) einen Extinktionskoeffizienten $E_{1cm}^{1\%}$ (280 nm) von $5,9\pm0,6$;

f) einen Kohlenhydratanteil von $2,4\pm0,94\%$ (g/100 g) (Mannose $0,3\pm0,2\%$, Galactose $0,4\pm0,2\%$ Xylose $0,1\pm0,04\%$, Glucose $0,2\pm0,1\%$, Glukosamin $1,0\pm0,2\%$, Neuraminsäure $0,4\pm0,2\%$) und

g) die folgende Aminosäurenzusammensetzung:

| Aminosäure | Reste pro 100 Reste (Mol%) | Variations- koeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

Zur Erläuterung der kennzeichnenden Merkmale des Gewebsproteins sie folgendes ausgeführt:

Die elektrophoretische Beweglichkeit wurde in der Mikromodifikation mit dem Gerat mit der Handelsbezeichnung Microzone R200 von Beckman Instruments auf Zelluloseazetatfolien (Firma Sartorius) unter Verwendung von Natriumdiäthylbarbiturat-Puffer, pH 8,6, bestimmt.

Der isoelektrische Punkt wurde mit einer Säule (440 ml) der Firma LKB, Stockholm, ermittlt. Das Ampholin®-Gemisch hatte einen pH-Bereich von 4,0 bis 6,0.

Der Sedimentationskoeffizient wurde in einer analytischen Ultrazentrifuge der Firma Beckman (Handelsbezeichnung Spinco-Apparatur, Modell E) bei 60.000 UpM in Doppelsektorzellen mit Hilfe der UV-

2

## EP 0 123 307 B1

Scanner-Technik bei 280 nm bestimmt. Als Lösungsmittel diente ein 0,05 mol/l Phosphatpuffer (pH 6,8), der 0,2 mol/l NaCl enthielt. Die Proteinkonzentration wurde auf eine optische Dichte von etwa 3 eingestellt. Der Sedimentationskoeffizient wurde auf die Basis von Wasser bei 20°C umgerechnet.

Zur Bestimmung der Molekulargewichte im SDS-haltigen Polyacrylamidgel wurde ein Gel mit 7,5 g/100 ml Polyacrylamid (PAA), das 0,1 g/100 ml Natriumdodecylsulfat (Sds) enthielt, verwendet. Als Vergleichssubstanz dienten humanes Plazentalaktogen (HPL) und Human-Albumin sowie dessen Aggregate.

Zur Bestimmung des Extinktionskoeffizienten wurde 1 mg Substanz in destilliertem Wasser zu 1 ml Lösung gelöst.

Die Kohlenhydrate wurden wie folgt bestimmt: Nach Hydrolyse der glykosidischen Bindungen wurden die freigesetzten Neutralzucker als Boratkomplexe über eine Anionenaustauschersäule getrennt (Y. C. Lee et al., Anal. Biochem. 27 (1969), 567), im Eluat durch Zumischen von Cu(I)-bicinchoninatreagenz (K. Mopper und M. Gindler, Anal. Biochem. 56 (1973), 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI (1963), 463—465) ermittelt worden.

Die Aminosäurenanalyse wurde nach S. Moore, D. H. Spackman, W. H. Stein, Anal. Chem. 30 (1958), 1185, unter Verwendung des Flüssigkeitschromatographen mit der Handelsbezeichnung Multichrom B der Firma Beckman durchgeführt. Cystin wurde nach Oxydation des Proteins mit Perameinsäure (S. Moore et al., Anal. Chem. 30 (1958), 1185) und nachfolgender Chromatographie (S. Moore, J. Biol. Chem. 238 (1963), 235) als Cysteinsäure bestimmt. Der Tryptophangehalt ist mit der direkten photometrischen Bestimmung nach H. Edelhoch, Biochemistry 6 (1967), 1948, ermittelt worden.

Bei der Untersuchung von Extrakten aus verschiedenen menschlichen Organen wurde $PP_4$ in größerer Konzentration in Plazenta, Magen, Blase, Niere, Nebenniere Haut und Milz mit immunchemischen Methoden nachgewiesen. Extrakte aus anderen Organen des Menschen wie Herz, Lunge, Leber, Colon, Rektum und Uterus enthielten dieses Protein nicht oder in wesentlichen geringeren Mengen. In geringer Konzentration kommt $PP_4$ im Lysat aus menschlichen Erythrozyten vor (etwa 0,6 mg $PP_4$ pro 100 ml kompakten gewaschenen Erythrozyten). Mit $PP_4$ immunchemisch identische oder weitgehend verwandte Protein konnten auch in Extrakten aus Plazenten von Affen sowie von Kuh und Schaf nachgewiesen werden.

Zur Isolierung von $PP_4$ können demnach Organe und Zellen des Menschen oder aber auch von Tieren, in denen dieses Protein vorkommt, verwendet werden. Insbesondere eignen sich dafür ausgewachsene menschliche Plazenten, die in reichlicher Menge anfallen und das Protein in genügend großer Konzentration enthalten.

Eine ausgewachsene menschliche Plazenta enthält im Durchschnitt etwa 50 mg $PP_4$. Das in der Plazenta enthaltene $PP_4$ geht bei der Extraktion des Organs mit verdünnten Salz- oder Pufferlösungen, beispielsweise mit physiologischer NaCl-Lösung, nur zum Teil in Lösung (etwa 2 bis 5 mg pro Plazenta). Der Hauptanteil des Proteins scheint aber im Gewebe an Membranen assoziiert zu sein und geht erst bei Anwendung solubilisierender Agentien, beispielsweise nicht-ionischer Detergenzien wie Polyethylenglycol-p-isooctylphenylether (Triton® X-100), in Lösung. Zur Gewinnung von $PP_4$ kann demnach sowohl der mit verdünnten Salzlösungyn erhaltene Proteinextrakt aus Plazenten als auch der nach Auswaschen der löslichen Bestandteile aus dem Geweberückstand durch Solubilisieren mit Triton® X-100 gewonnene Proteinextrakt von Plazenten verwendet werden. Die nach beiden Extraktionsmethoden gewonnenen Proteine sind in ihren physikalisch-chemischen und immunchemischen Eigenschaften identisch.

$PP_4$ hat folgende Eigenschaften, die sich in einem Verfahren zu seiner Isolierung verwenden lassen, indem diesen Eigenschaften entsprechende Maßnahmen getroffen werden:

1) Mit Ammoniumsulfat wird es bei pH 7,0 und 40—70% Sättigung aus wässrigen Lösungen gefällt;

2) Mit wasserlöslichen Acridinbasen, z.B. 2-Äthoxy-6,9-diaminoacridinlactat (Rivanol®) wird es bei pH-Werten zwischen 4—9 und einer Konzentration der Base von 0,2 bis 0,8 g/100 ml präzipitiert. Bei einem pH-Wert von 6,0 und einer Rivanolkonzentration von 0,4 g/100 ml fällt es zum Teil aus;

3) Bei einer elektrophoretischen Auftrennung wird es bei pH 8,6 im Bereich zwischen $\alpha_1$ und $\alpha_2$ Globulinen gefunden;

4) Bei der isoelektrischen Fokussierung erscheint es im pH-Bereich von 4,7 bis 5,0;

5) Bei einer Gelfiltration mit Sephadex® verhält es sich wie Proteine mit Molekulargewichten von 20,000 bis 50,000;

6) Es wird in verdünnten Salzlösungen bei einer Leitfähigkeit von etwa 0—2 mS an Kaolin, beispielsweise mit der Handelsbezeichnung Bolus weiß, Fa. Merck, Darmstadt, adsorbiert und dann mit stärker konzentrierten Salzlösungen, beispielsweise 0,1 mol/l Phosphatpuffer, pH 6,8, wieder davon eluiert werden;

7) Es läßt sich an schwach basische Ionenaustauscher, beispielsweise DEAE-Cellulose oder DEAE-Sephadex®, bei einer Leitfähigkeit von etwa 0—2 mS und einem pH-Wert von etwa pH 7 bis 9 binden und kann mit stärker konzentrierten Salzlösungen (1—5 g/100 ml NaCl-Lösungen) eluiert werden;

8) Es kann aus einer wässrigen Lösung durch Immunadsorption angereichert und isoliert werden.

Gegenstand der Erfindung ist auch ein Verfahren zur Gewinnung des PP4, dadurch gekennzeichnet,

3

EP 0 123 307 B1

daß aus einem aus Organen, die dieses Protein enthalten, mit verdünnten Salz- oder Pufferlösungen gewonnenen Extrakt das Protein PP4 mit einer wasserlöslichen Acridin- oder chinolin base bei einem pH-Wert zwischen 4 und 9 und einer Konzentration der Base von 0,2—0,8 g/100 ml gefällt wird, daß Begleitproteine durch Zugabe von 25 g Ammoniumsulfat auf 100 ml Lösung zum Teil ausgefällt werden, daß die Lösung des PP4 einer Gelfiltration oder Ultrafiltration, wobei Proteine im Molekulargewichtsbereich von 20.000 bis 50.000 gewonnen werden, unterworfen wird, daß das PP4 an Kaolin adsorbiert und eluiert wird, und daß das Eluat einer weiteren Gelfiltration und einer inversen Immunadsorption unterworfen wird.

Neben Ammoniumsulfat können auch andere in der präparativen Biochemie üblicherweise eingesetzten Neutralsalze zur Ausfällung des PP4 verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten, seiner Ladung, wie seinem Molekulargewicht sind zur Isolierung des Proteins auch andere Maßnahmen brauchbar, die geeignet sind, ein Protein mit den angegebenen Eigenschaften von anderen Proteinen abzutrennen.

Hierzu können die verschiedenen Methoden der präparativen Elektrophorese, der isoelektrischen Fokussierung, der Gelfiltration oder Ultrafiltration oder auch die Eigenschaft des PP4, an schwach basische Ionenaustauscher gebunden und hiervon wieder eluiert werden zu können, verwendet werden.

Insbesondere aber ist die spezielle Eigenschaft von PP4, in verdünnten Pufferlösungen an Kaolin adsorbiert zu werden und mit starken Pufferlösungen hiervon wieder eluiert werden zu können, zur Isolierung dieses Proteins hervorragend geeignet.

Durch eine zweckmäßige Kombination der genannten Maßnahmen, die eine Anreicherung des PP4 oder eine Abtrennung dieses Proteins von anderen Proteinen bewirken, kann das PP4 isoliert werden.

Gegenstand der Erfindung ist besonders ein Verfahren zur Gewinnung des PP4, dadurch gekennzeichnet, daß man Organe, die dieses Protein enthalten, zerkleinert und mit physiologischer Salzlösung wäscht, bis alle löslichen Bestandteile entfernt sind, den Geweberückstand dann mit einem solubilisierenden Agens, vorzugsweise einer Lösung eines nichtionischen Detergenzes oder einer 3 mol KSCN und 6 mol Harnstoff pro 1 enthaltenden Lösung extrahiert und in dem erhaltenen Extrakt nach Dialyse das PP4 mittels Immunadsorption anreichert, von höhermolekularen Proteinen durch Gelfiltration abtrennt und restliche Verunreinigungen mittels inverser Immunadsorption entfernt.

Die angegebenen Maßnahmen sind zur Anreicherung von PP4 keinesfalls alle zwingend und müssen auch nicht in der angegebenen Reihenfolge durchgeführt werden.

Bei der Isolierung von PP4 aus dem verdünnten Salzextrakt von menschlichen Plazenten hat es sich als zweckmäßig erwiesen, die Proteine im Extrakt zunächst mit einer wasserlöslichen Acridinbase und Ammoniumsulfat zu fraktionieren und anschließend durch Gelfiltration weiter anzureichern, bevor an Kaolin adsorbiert wird (vgl. Beispiel 1).

Bei der Isolierung von PP4 aus der durch Extraktion des Plazentengewebes mit Detergenz gewonnenen Proteinfraktion hat es sich als vorteilhaft erwiesen, PP4 zunächst durch einen Immunadsorptionsschritt anzureichern und zur weiteren Reinigung eine Gelfiltration anzuschließen (vgl. Beispiel 2).

Zum Nachweise und zur Bestimmung des PP4, etwa in einer Fraktion aus einer Trennoperation, können neben den angegebenen Parametern auch immunchemische Methoden dienen, da PP4 antigene Eigenschaften hat.

Ein für diesen Zweck brauchbares Antiserum kann folgendermaßen gewonnen werden: Durch Immunisieren von Kaninchen mit der mittels Detergenz solubilisierten Plazentagewebsproteinfraktion wird ein polyvalentes Antiserum erhalten, das unter anderem auch Antkörper gegen PP4 enthält.

Dieses Antiserum kann einerseits zum immunologischen Nachweis des $PP_4$, andererseits zur Herstellung eines Immunadsorbens dienen, das zur Anreicherung und Isolierung von $PP_4$ eingesetzt werden kann.

Mit Hilfe des nach Beispiel 1 der vorliegenden Anmeldung gewonnenen gereinigten $PP_4$ lassen sich durch Immunisieren von Tieren nach bekannten Methoden monospezifische Antiserum herstellen.

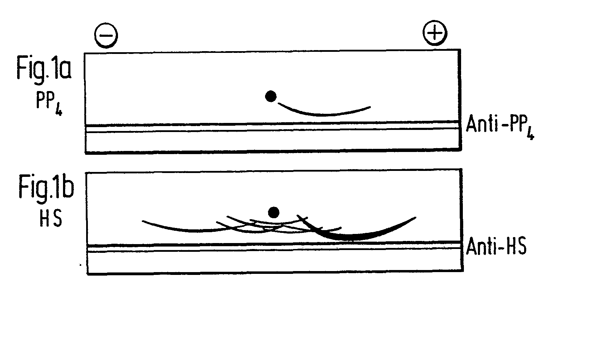

Abbildung 1a zeigt die immunologische Reaktion von $PP_4$ mit einem spezifischen Antiserum vom Kaninchen nach Auftrennung im elektrischen Feld in Agar-haltigem Gel.

Abbildung 1b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von $PP_4$ können auch die Geldiffusionstechnik nach Ouchterlony (vgl. Schultze and Heremans, Molecular Biology of Human Proteins, Vol. 1, pg. 134) oder, wenn notwendig, auch empfindlichere Methoden, wie Radioimmunoassays oder Enzymimmunoassays, herangezogen werden.

Der Nachweis und die Bestimmung von $PP_4$ haben diagnostische Bedeutung. $PP_4$ ist ein Gewebsprotein, das nur in bestimmten Organen in größerer Konzentration vorkommt. Bei einer Erkrankung dieser Organe kann infolge eines vermehrten Zellzerfalls die Konzentration des Gewebsproteins $PP_4$ im Serum oder in anderen Körperflüssigkeiten, beispielsweise im Urin, der Patienten über die Norm ansteigen. Der Nachweis und die Bestimmung von $PP_4$ in Körperflüssigkeiten können daher zur Erkennung von Erkrankungen dieser Organe oder auch als Marker zur Überwachung des Krankheitsverlaufs und zur Kontrolle der Therapie eingesetzt werden.

4

PP$_4$ kann also verwendet werden, um Antiseren herzustellen, die dazu dienen können, PP$_4$ nachzuweisen und zu bestimmen.

Die Erfindung wird an nachstehenden Beispielen erläutert:

Beispiel 1

A) Extraktion der Plazenten und fraktionierung des Extraktes mit einer acridinbase und ammoniumsulfat

1.000 kg tiefgefrorene menschliche Plazenten wurden im Schneidmischer zerkleinert und mit 1.000 Liter einer 0,4%igen (g/100 ml) Kochsalzlösung extrahiert. Der Extrakt wurde nach Abtrennung des Geweberückstandes durch Zentrifugation mit 20%iger (g/100 ml) Essigsäure auf pH 6,0 eingestellt und unter Rühren mit 200 Liter einer 3%igen (g/100 ml) Lösung von 2-Äthoxy-6,9-diaminoacridin-lactat (Rivanol®, Hoechst AG) versetzt. Der Niederschlag wurde durch Zentrifugation abgetrennt, mit 500 Liter einer 2,5%igen (g/100 ml) NaCl-Lösung versetzt und 4 Stunden gerührt. Das abgeschiedene Chlorid des 2-Äthoxy-6,9-diaminoacridins wurde abzentrifugiert. Aus dem Überstand wurde ein Teil der Begleitproteine durch Zusatz von 25% (g/100 ml) Ammoniumsulfat abgeschieden. Das Protein PP$_4$ blieb zur Hauptsache in Lösung; es wurde daraus durch weitere Zugabe von Ammoniumsulfat (20g/100 ml) präzipitiert und dann abzentrifugiert. Es wurden dabei etwa 3 kg einer feuchten Paste, die im Nachfolgenden als Fraktion A beziechnet wird, erhalten.

B) Gelfiltration an Sephadex g-150

500 g der Fraktion A wurden in Wasser gelöst, zur Entfernung des noch vorhandenen Rivanols mit etwa 2,5 g Bentonit A (Fa. Erbslöh & Co., Geisenheim/Rh.) versetzt und nach Zentrifugation gegen einen 0,01 mol/l Tris-HCl-Puffer (pH 8,0), der 0,05% (g/100 ml) NaN$_3$ enthielt (Pufferlösung I), dialysiert. Die zurückbleibende Lösung wurde auf eine mit Sephadex® G-150 gefüllt Säule (20×100 cm) aufgetragen und mit Pufferlösung I eluiert. Die Eluate mit den niedermolekularen Proteinen (MG 10.000 bis 60.000) wurden vereinigt und zur Fällung der Proteine mit 45% (g/100 ml) Ammoniumsulfat versetzt. Der Niederschlag wurde abzentrifugiert (Fraktion B).

C) Adsorption an kaolin und elution

Fraktion B wurde in etwa 100 ml Wasser gelöst und gegen Pufferlösung I dialysiert. Je 150 ml dieser Lösung wurden mit 15 g Kaolin (Bolus weiß, Fa. Merck, Darmstadt) versetzt und 1 Stunde bei 20°C gerührt. PP$_4$ wurde dabei an Kaolin gebunden, während die anderen Proteine zur Hauptsache in der Lösung zurückblieben. Nach Zentrifugation wurde das Kaolin zweimal kurz mit Pufferlösung I gewaschen und anschliessend zweimal hintereinander mit je 150 0,1 mol/l Natriumphosphatpuffer, pH 6,8, eluiert, wobei PP$_4$ wieder in Lösung ging. Nach Abtrennung des Kaolins durch Zentrifugation wurden die Eluate vereinigt und auf einem Ultrafilter auf 10—20 ml eingeengt.

D) Hochreinigung

Das durch Adsorption an Kaolin gewonnen Präparat hatte eine Reinheit von etwa 90%. Die Hauptmenge der noch vorhandenen Verunreinigungen ließ sich durch Gelfiltration an einer Ultrogel AcA-44 Säule (4,5×100 cm) unter Verwendung einer 0,1 mol/l Tris-HCl-Pufferlösung von pH 8, die 1 mol/l NaCl und 0,1% (g/100 ml) Natriumazid enthielt (Pufferlösung II), abtrennen. Die restlichen noch in Spuren vorhandenen Serumproteine wurden durch inverse oder negative Immunadsorption entfernt, d.h. mit Hilfe von trägergebundenen Antikörpern vom Kaninchen, die gegen die Proteine des menschlichen Serums gerichtet waren. Das aus diese Weise gewonnene PP$_4$ hatte eine Reinheit von <99%. Die PP$_4$-Lösung wurde gegen Wasser dialysiert und anschließend lyophilisiert. Ausbeute etwa 30 mg PP$_4$ aus 500 g Paste der Fraktion A.

Beispiel 2

A) Zerkleinerung und Wachng der plazenten

Zur Isolierung des membran-assoziierten Teils von PP$_4$ wurden reife menschliche Plazenten, wie sie bei einer Entbindung anfallen, im gefrorenen Zustand mit einem Schneidmischer zerkleinert und in dieser Form bei −20°C bis zur Verwendung aufbewahrt. Durch Waschung mit physiologischer NaCl-Lösung wurden zunächst alle löslichen Gewebsproteine entfernt. Dazu wurfen 500 g des zerkleinerten Plazentengewebes mit 700 ml NaCl-Lösung versetzt, kurz homogenisiert, dann mehrere Stunden bei 4°C gerührt und schließlich zentrifugiert. Der Überstand wurde verworfen, der Rückstand erneut mit 700 ml NaCl-Lösung mehrere Stunden gerührt und wieder zentrifugiert. Dieser Waschvorgang wurde insgesamt 6 mal wiederholt. Auf diese Weise wurde das Plazentagewebe von löslichen Bestandteilen weitgehend befreit.

B) Extraktion des plazentagewebes mit detergenz

Zur Solubilisierung der Membran-assoziierten Antigene wurde der Geweberückstand nach der Waschung hintereinander dreimal mit je 700 ml einer 2%igen Lösung von Triton® X-100 in Wasser extrahiert, wobei jeweils 20 Stunden bei 4°C gerührt und dann abzentrifugiert wurde. Die Extrakte sind zunächst gegen Wasser und dann gegen einen 0,1 mol/l Tris-HCl-Puffer (pH 8,0), der 1 mol/l NaCl und 0,1% (g/100 ml) Natriumazid enthielt (Pufferlösung II), dialysiert worden. Nach der Dialyse wurden die Lösungen auf einem Ultrafilter (Fa. Amicon) unter Verwendung von PM-10 Membranen auf jeweils etwa 200 ml eingeengt. Die Extrakte aus dem Rückstand von 500 g Plazentagewebe enthielten im Durchschnitt insgesamt etwa 25 mg $PP_4$ (Fraktion 2A).

C) Anreicherung von PP4 durch immunadsorption
1) Herstellung des immunadsorbens

Durch Immunisieren von Kaninchen mit der solubilisierten Plazentaprotein (Fraktion 2A) wurden polyvalente Antiseren hergestellt. Sie enthielten Antikörper sowohl gegen $PP_4$ als auch gegen andere mit Detergenz lösliche Antigene. 350 ml eines solchen Antiserumpools wurden gegen 0,02 mol/l Phosphatpuffer (pH 7,0) dialysiert und zur Abtrennung der Immunglobuline mit dem gleichen Puffer an DEAE-Zellulose chromatographiert. Die Immunglobulinfraktion im Durchlauf (3,63 g Protein) wurde dann mit 363 g besonders gereinigter Agarose in Kugelform (Sepharose® von Pharmacia, Uppsala, Schweden), die mit 45,3 g Bromycan aktiviert worden war, umgesetzt und so kovalent an diesen Träger gebunden. Das Verfahren wurde beschrieben von Axen et al., Nature 214 (1967) 1302. Mit Hilfe eines auf diese Weise hergestellten Immunadsorbens konnte $PP_4$ zusammen mit anderen solubilisierten Antigenen aus der Plazentafraktion 2A weiter angereichert werden.

2) Durchführung der Immunadsorption

Das Immunadsorbens wurde in Pufferlösung II suspendiert, in eine Chromatographiesäule gefüllt $(5,0\times20$ cm) und mit Pufferlösung II nachgespült. Dann wurde Fraktion 2A auf die Säule aufgetragen, wobei $PP_4$ und andere solubilisierte Antigene immunadsorption gebunden wurden. Die Säule wurde dann gründlich mit Puffer II gespült. Anschließend sind die adsorbierten Proteine mit etwa 600 ml 6 mol/l Harnstoff-Lösung von der Säule eluiert worden. Die $PP_4$-haltigen Eluate wurden gegen Pufferlösung II dialysiert und mit einem Ultrafilter auf etwa 20 ml eingeengt. Das Adsorbens in der Säule wurde unmittelbar nach der Elution der Proteine wieder mit Pufferlösung II neutralisiert und gründlich gewaschen. Es ist dann erneut zur immunadsorptiven Bindung der solubilisierten Antigene eingesetzt worden.

D) Abtrennung von PP4 durch gelfiltration

Die Abtrennung von $PP_4$ von den anderen immunadsorptiv gebundenen Antigenen gelang durch Gelfiltration an Acrylamidagarose AcA-34 (LKB, Stockholm). Dazu wurden durch Immunadsorption gewonnene Fraktionen vereinigt, auf 60 ml eingeengt und unter Verwendung der Pufferlösung II an einer AcA-34 Säule $(5\times110$ cm) chromatographiert. $PP_4$ wurde dabei deutlich von den anderen solubilisierten Membranantigenen abgetrennt, die zur Hauptsache ein höheres Molekulargewicht (mehr als 100.000) besitzen und daher schneller als $PP_4$ durch die Säule wandern. Die Fraktionen, welche die Hauptmenge $PP_4$ enthielten, wurden vereinigt, und im Ultrafilter auf 10—20 ml eingeengt.

E) Hochreinigung

Das bei der Gelfiltration erhaltene Präparat war noch durch geringe Mengen von Serumproteinen, insbesondere durch Albumin, verunreinigt. Diese konnten durch inverse Immunadsorption, d.h. mit Hilfe von trägergebundenen Antikörpern gegen die noch als Begleitproteine vorhandenen Seru1proteine entfernt werden.

**Patentansprüche**

1. Protein $PP_4$, gekennzeichnet durch
a) eine elektrophoretische Beweglichkeit im Bereich zwischen der von $\alpha_1$- und $\alpha_2$-Globulinen;
b) einen isoelektrischen Punkt von $4,85\pm0,15$;
c) einen Sedimentationskoeffizienten $s^0_{20,w}$ von $3,3\pm0,2$ S;
d) ein im Natriumdodecylsulfat (SDS)-haltigen Polyacrylamidgel bestimmtes Molekulargewicht von $35.000\pm5.000$;
e) einen Extinktionskoeffizienten $E^{1\%}_{1cm}$ (280 nm) von $5,9\pm0,6$;
f) einen Kohlenhydratanteil von $2,4\pm0,94\%$ (g/100 g) (Mannose $0,3\pm0,2\%$, Galactose $0,4\pm0,2\%$, Xylose $0,1\pm0,04\%$, Glukose $0,2\pm0,1\%$, Glukosamin $1,0\pm0,2\%$, Neuraminsäure $0,4\pm0,2\%$) und
g) die folgende Aminosäurenzusammensetzung:

| Aminosäure | Reste pro 100 Reste (Mol %) | Variations-koeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

2. Verfahren zur Gewinnung des Protein $PP_4$ nach Anspruch 1, dadurch gekennzeichnet, daß aus einem aus Organen, die dieses Protein enthalten, mit verdünnten Salz- oder Pufferlösungen gewonnenen Extrakt das Protein $PP_4$ mit einer wasserlöslichen Acridin -oder Chinolin base bei einem pH-Wert zwischen 4 und 9 und einer Konzentration der Base von 0,2—0,8 g/100 ml gefällt wird, daß Begleitproteine durch Zugabe von 25 g Ammoniumsulfat auf 100 ml Lösung zum Teil ausgefällt werden, daß die Lösung des $PP_4$ einer Gelfiltration oder Ultrafiltration, wobei Proteine im Molekulargewichtsbereich von 20.000 bis 50.000 gewonnen werden, unterworfen wird, daß das $PP_4$ an Kaolin adsorbiert und eluiert wird, und daß das Eluat einer weiteren Gelfiltration und einer inversen Immunadsorption unterworfen wird.

3. Verfahren zur Gewinnung des Proteins $PP_4$ nach Anspruch 1, dadurch gekennzeichnet, daß man Organe, die dieses Protein enthalten, zerkleinert und mit physiologischer Salzlösung wäscht, bis alle löslichen Bestandteile entfernt sind, den Geweberückstand dann mit einem solubilisierenden Agens, vorzugsweise mit einer Lösung eines nicht-ionischen Detergenzes oder einer 3 mol KSCN oder 6 mol Harnstoff pro 1 enthaltenden Lösung, extrahiert und in dem erhaltenen Extrakt nach Dialyse das $PP_4$ mittels Immunadsorption anreichert, von höhermolekularen Proteinen durch Gelfiltration abtrennt und restliche Verunreinigungen mittels inverser Immunadsorption entfernt.

4. Verwendung des Proteins $PP_4$ zur Herstellung von Antiseren zum Nachweis und zur Bestimmung von $PP_4$ in Körperflüssigkeiten, um Erkrankungen bestimmter Organe zu erkennen, als "Marker" einen Krankheitsverlauf zu überwachen oder eine Therapie zu kontrollieren.

7

**Revendications**

1. Protéine $PP_4$, caractérisée par

a) une mobilité, à l'électrophorèse, qui se situe entre celles des globulines $\alpha_1$ et $\alpha_2$;

b) un point isoélectrique de 4,85±0,15;

c) un coefficient de sédimentation $s^0_{20,w}$ de 3,3±0,2 S;

d) un poids moléculaire déterminé sur un gel de polyacrylamide renfermant du dodécylsulfonate de sodium (SDS), de 35000±5000;

e) un coefficient d'extinction $E_1{}^{1\%}_{cm}$ (280 nm) de 5,9±0,6;

f) une proportion d'hydrates de carbone de 2,4±0,94% (g/100 g) (mannose 0,3±0,2%, galactose 0,4±0,2%, xylose 0,1±0,04%, glucose 0,2±0,1%, glucosamine 1,0±0,2%, acide neuraminique 0,4±0,2%) et

g) la composition en acides aminés suivante:

| Acide aminé | Restes pour cent restes (mol%) | Coeff. de variation |
|---|---|---|
| Lysine | 6,95 | 1,14 |
| Histidine | 0,97 | 17,4 |
| Arginine | 5,44 | 1,77 |
| Acide aspartique | 11,41 | 1,68 |
| Thréonine | 6,78 | 2,40 |
| Sérine | 6,21 | 2,26 |
| Acide glutamique | 12,25 | 0,43 |
| Proline | 1,96 | 6,20 |
| Glycine | 6,68 | 3,83 |
| Alanine | 7,92 | 1,67 |
| Cystine 1/2 | 0,77 | 19,5 |
| Valine | 5,34 | 3,80 |
| Méthionine | 1,98 | 6,00 |
| Isoleucine | 5,21 | 2,23 |
| Leucine | 11,50 | 0,45 |
| Tyrosine | 3,55 | 4,21 |
| Phénylalanine | 4,07 | 3,77 |
| Tryptophane | 0,93 | 23,9 |

2. Procédé d'obtention de la protéine $PP_4$ selon la revendication 1, caractérisé en ce que l'on précipite, à partir d'un extrait d'organes renfermant cette protéine obtenu avec des solutions salines ou tamponnées diluées, la protéine $PP_4$, à l'aide d'une base acridine ou quinoléine hydrosoluble à un pH compris entre 4 et 9 et à une concentration de la base de 0,2 à 0,8 g/100 ml, que l'on précipite partiellement les protéines associées par addition de 25 g de sulfate d'ammonium pour 100 ml de solution, que l'on soumet la solution de protéine $PP_4$ à une filtration sur gel ou à une ultrafiltration, ce qui permet d'obtenir des protéines dont la masse moléculaire se situe entre 20000 et 50000, que l'on adsorbe ladite protéine sur kaolin puis que l'on procède à son élution, et que l'on soumet l'éluat à une autre filtration sur gel et à une immunoadsorption inverse.

3. Procédé d'obtention de la protéine $PP_4$, caractérisé en ce que l'on broie des organes contenant cette protéine, et qu'on les lave avec une solution saline physiologique jusqu'à ce que tous les constituants solubles soient éliminés, en ce que l'on extrait le résidu tissulaire avec un agent solubilisant, de préférence une solution d'un détergent non-ionique ou une solution contenant 3 mol de KSCN ou 6 mol d'urée par 1, et que l'on enrichit par immunoadsorption PP₄ dans l'extrait obtenu après dialyse, qu'on la sépare par filtration sur gel des protéines de masses moléculaires plus élevées, et que l'on élimine les impuretés restantes par immunoadsorption inverse.

4. Utilisation de la protéine PP₄ pour la fabrication d'antisérums destinés à permettre l'identification et le dosage de PP₄ dans les liquides de.l'organisme, afin de diagnostiquer les affections de certains organes, et qui agissent comme "marqueurs" pour suivre l'évolution d'une maladie ou pour contrôler une thérapeutique.

**Claims**

1. Protein PP₄, which has the following characterisics
a) an electrophoretic mobility in the range between that of $\alpha_1$- and $\alpha_2$- globulins;
b) an isoelectric point of 4.85±0.15;
c) a sedimentation coefficient $S_{20,w}^0$ of 3.3±0.2 S;
d) a molecular weight determined in polyacrylamide gel containing sodium dodecylsulfate (SDS) of 35,000±5,000;
e) an extension coefficient $E_{1cm}^{1\%}$ (280 nm) of 5.9±0.6;
f) a carbohydrate content of 2.4±0.94% (g/100 g) (mannose 0.3±0.2%, galactose 0.4±0.2%, xylose 0.1±0.04%, glucose 0.2±0.1%, glucosamine 1.0±0.2% and neuraminic acid 0.4±0.2%) and
g) the following amino acid composition:

| Amino acid | Residues per 100 residues (mol-%) | Coefficient of variation |
|---|---|---|
| Lysin | 6.95 | 1.14 |
| Histidine | 0.97 | 17.4 |
| Arginine | 5.44 | 1.77 |
| Aspartic acid | 11.41 | 1.68 |
| Threonine | 6.78 | 2.40 |
| Serine | 6.21 | 2.26 |
| Glutamic acid | 12.25 | 0.43 |
| Proline | 1.96 | 6.20 |
| Glycine | 6.68 | 3.83 |
| Alanine | 7.92 | 1.67 |
| Cystine 1/2 | 0.77 | 19.5 |
| Valine | 5.34 | 3.80 |
| Methionine | 1.98 | 6.00 |
| Isoleucine | 5.21 | 2.23 |
| Leucine | 11.50 | 0.45 |
| Tyrosine | 3.55 | 4.21 |
| Phenylalanine | 4.07 | 3.77 |
| Tryptophan | 0.93 | 23.9 |

2. A process for isolating protein PP₄ as claimed in Claim 1, which comprises precipitating the protein PP₄ with a water-soluble acridine or quinoline base at a pH between 4 and 9, and at a concentration of the base of 0.2—0.8 g/100 ml, from an extract isolated with dilute salt or buffer solutions from organs which contains this protein, precipitating some of the concomitant proteins by adding 25 g of ammonium sulfate per 100 ml of solution, subjecting the $PP_4$ solution to a gel filtration or ultrafiltration which entails proteins in the molecular weight range of 20,000 to 50,000 being isolated, adsorbing the $PP_4$ on kaolin and eluting it, and subjecting the eluate to further gel filtration and inverse immunoadsorption.

3. A process for isolating protein $PP_4$ as claimed in Claim 1, which comprises organs which contain this protein being comminuted and washed with physiological saline until all soluble constituents have been removed, the tissue residue then being extracted with a solubilizing agent, preferably with a solution of a non-ionic detergent or a solution which contains 3 mol of a KSCN or 6 mol of urea per 1, and the $PP_4$ being concentrated, by means of immunoadsorption, in the resulting extract after dialysis, being separated from higher molecular weight proteins by gel filtration, and remaining impurities being removed by inverse immunoadsorption.

4. The use of the protein $PP_4$ for the preparation of antisera for detecting and determining $PP_4$ in body fluids, in order to diagnose diseases of particular organs, as a "marker" to monitor the progress of a disease or to check therapy.

Fig.1a
PP₄

Fig.1b
HS